# FASCICULE DE BREVET EUROPEEN

(11) **EP 1 151 741 B1**
(45) Date de publication et mention de la délivrance du brevet: **25.10.2006**
(21) Numéro de dépôt: 01401125.8
(22) Date de dépôt: 30.04.2001
(51) Int. Cl.: A61K 8/11, A61Q 5/00, A61Q 17/04, A61Q 19/02, A61Q 19/04, A61Q 19/06, A61Q 19/08, A61K 9/50

(54) **Microcapsules à coeur aqueux contenant au moins un principe actif cosmétique ou dermatologique hydrosoluble et compositions cosmétiques ou dermatologiques les contenant**
Mikrokapsel mit wasserhaltigem Kern mit mindestens einem wasserlöslischen kosmetischen oder dermatologischen Wirkstoff und kosmetische oder dermatologische Zubereitungen, diese enthaltend
Microcapsules with an aqueous core containing at least an active water-soluble cosmetic or dermatologic ingredient and cosmetic or dermatologic compositions containing these microcapsules

(30) Priorité: 05.05.2000 FR 0005756
(43) Date de publication de la demande: 07.11.2001
(73) Titulaire: L'ORÉAL, 75008 Paris (FR)
(72) Inventeur: Biatry, Bruno, 94300 Vincennes (FR); Lheureux, Eric, 91230 Montgeron (FR)
(74) Mandataire: Casalonga, Axel

(56) Documents cités:
- EP-A- 0 190 833
- EP-A- 0 273 890
- EP-A- 0 316 054
- DE-A- 19 802 644
- FR-A- 2 245 335
- GB-A- 1 297 476
- US-A- 4 107 071
- US-A- 5 154 762
- US-A- 5 238 714
- US-A- 5 589 194

## Description

La présente invention concerne des compositions cosmétiques ou dermatologiques contenant des microcapsules à coeur aqueux contenant au moins un principe actif cosmétique ou dermatologique hydrosoluble et un procédé pour préparer de telles microcapsules.

La microencapsulation d'actifs cosmétiques ou dermatologiques en vue d'une meilleure conservation et/ou d'une libération prolongée et contrôlée est connue.

Des procédés de microencapsulation et les principes auxquels ils font appel sont décrits en détail par exemple dans "Microencapsulation, Methods and Industrial Application", édité sous la direction de Benita, M. Dekker, 1996.

Tous ces procédés font appel à l'utilisation de polymères comme éléments constitutifs de la paroi de la microparticule qui va isoler l'actif du milieu extérieur. Ces microparticules permettent d'encapsuler des taux plus élevés d'actifs hydrosolubles que d'autres systèmes vésiculaires tels que les liposomes, qui sont des nanoparticules formées de doubles couches phospholipidiques entourant un coeur aqueux.

On a ainsi cherché à encapsuler différents actifs hydrophiles. Parmi ceux-ci, l'acide ascorbique présente un intérêt tout particulier, dans la mesure où son instabilité en milieu aqueux rend difficile sa formulation dans des compositions cosmétiques, alors qu'il présente de nombreuses propriétés bénéfiques.

Précisément, en raison de sa structure chimique (α-cétolactone), l'acide ascorbique est très sensible à certains paramètres de l'environnement comme la lumière, l'oxygène ou l'eau. Il s'ensuit donc une dégradation rapide de l'acide ascorbique formulé en présence de ces agents. Cette instabilité est susceptible de nuire à son efficacité alors qu'il constitue un actif de choix pour stimuler la synthèse du tissu conjonctif et notamment du collagène, renforcer les défenses du tissu cutané contre les agressions extérieures telles que les rayonnements ultraviolets et la pollution, compenser la déficience en vitamine E de la peau, dépigmenter la peau et piéger les radicaux libres. Ces propriétés en font en particulier un excellent candidat comme actif cosmétique ou dermatologique pour lutter contre le vieillissement de la peau ou prévenir celui-ci.

Parmi les techniques permettant d'encapsuler des principes actifs hydrophiles tels que l'acide ascorbique, sous forme dissoute, dispersée ou pulvérulente, on peut citer la coacervation (JP-8325117, JP-5285210 et US-4 460 563), le séchage par nébulisation *(spray drying)* (US 5 767 107), la fluidisation en lit d'air (WO 95/27488 et EP 0 600 775), ou la polymérisation interfaciale (JP 1043343, WO 91/01801 et WO 94 23832).

Le séchage par nébulisation et la fluidisation en lit d'air ont pour principal inconvénient d'encapsuler l'actif sous forme de poudre, c'est-à-dire à l'état solide. Or, il est souvent essentiel dans le domaine cosmétique ou dermatologique, d'encapsuler l'actif sous forme dissoute dans un milieu aqueux pour assurer sa biodisponibilité cutanée immédiate.

Les techniques de coacervation ou de polymérisation interfaciale ne sont pas appropriées pour la microencapsulation de principes actifs cosmétiques ou dermatologiques hydrosolubles car elles impliquent généralement l'utilisation de réactifs polymérisables ou bifonctionnels toxiques pour la peau et susceptibles de réagir avec l'actif à encapsuler et de le désactiver.

Un autre procédé de microencapsulation a également été utilisé pour encapsuler dans des microparticules des actifs hydrophiles à l'état solubilisé. Il s'agit de la technique dite "émulsion multiple-évaporation ou extraction de solvant", qui consiste à préparer une émulsion primaire eau-dans-huile par dispersion d'une solution aqueuse du principe actif dans une solution organique d'un polymère insoluble dans l'eau, avant de disperser, dans un second temps, cette émulsion primaire dans une phase aqueuse externe. Le solvant organique est ensuite éliminé par évaporation ou extraction.

Ce procédé est décrit dans les brevets US-3,523,906, US-3,523,907, EP 190 833 et WO 95/28149.

Les brevets US-3,523,906 et US-3,523,907 décrivent respectivement un procédé d'émulsion multiple-évaporation et un procédé d'émulsion multiple-extraction utilisant, comme polymères constitutifs de la paroi des microcapsules, des polymères ou copolymères vinyliques, des polycondensats tels que des polycarbonates, des polyesters, des polysulfonates, des polyuréthannes ou des polyamides, ou des polymères naturels tels que le caoutchouc naturel chloré ou des dérivés de cellulose.

La demande de brevet EP 190 833 décrit un procédé d'encapsulation de principes actifs hydrosolubles par émulsion multiple-évaporation de solvant utilisant des polymères biocompatibles insolubles dans l'eau, et en particulier des polymères à base d'acide lactique et d'acide glycolique.

La demande de brevet WO 95/28149 décrit la préparation, par un procédé d'émulsion multiple-évaporation, de microcapsules ayant une paroi constituée d'un copolymère de type poly(lactide-co-glycolide).

Or, la demanderesse a constaté que les polymères cités dans les documents ci-dessus n'étaient pas adaptés à la stabilisation de principes actifs soit parce que leur hydrolyse en milieu aqueux se traduit par la libération d'acides organiques modifiant le pH de la composition, soit parce qu'ils ne permettent pas d'obtenir des taux d'encapsulation satisfaisants.

Le problème à l'origine de la présente invention était par conséquent de mettre au point de nouvelles compositions aqueuses comprenant des microcapsules contenant, dans leur enveloppe, des polymères stables en milieu aqueux et permettant d'obtenir des taux d'encapsulation de principes actifs hydrosolubles satisfaisants.

Un autre problème - particulier à l'encapsulation de principes actifs cosmétiques - est lié à la présence d'eau dans les compositions cosmétiques.

En effet, la présence d'une phase aqueuse extérieure aux microcapsules nécessite une excellente imperméabilité de la paroi de celles-ci pour éviter le passage du principe actif dans la phase aqueuse externe qui annulerait tout le bénéfice de l'encapsulation. Ainsi, dans le domaine cosmétique, on recherche généralement des microcapsules à paroi imperméable qui ne libèrent le principe actif qu'après application, par exemple suite à la rupture ou à la biodégradation de l'enveloppe.

Ce problème ne se présente pas dans le domaine pharmaceutique où les microcapsules sont généralement conservées à l'état séché et doivent libérer le principe actif rapidement après avoir été mises en contact avec un milieu aqueux.

La demanderesse a découvert qu'il était possible de résoudre les problèmes énoncés ci-dessus, c'est-à-dire d'obtenir, avec des taux d'encapsulation satisfaisants, des microcapsules constituées d'une enveloppe renfermant une solution aqueuse d'au moins un principe actif cosmétique ou dermatologique stabilisé, en choisissant de manière appropriée les matériaux formant l'enveloppe parmi certains polymères et/ou certaines cires.

La présente invention a donc pour objet une composition cosmétique sous la forme d'un gel aqueux, d'une émulsion eau-dans-huile ou d'une émulsion huile-dans-eau, comprenant, dans un support physiologiquement acceptable, des microcapsules à coeur aqueux contenant au moins un principe actif cosmétique ou dermatologique hydrosoluble , instable, sensible aux conditions physico-chimiques environnantes, et à enveloppe polymérique et/ou cireuse constituée
- d'au moins un polymère choisi parmi la polycaprolactone, le poly(3-hydroxybutyrate), le poly(éthylène adipate), le poly(butylène adipate), les esters de cellulose et d'au moins un acide carboxylique en C₁₋₄, de préférence des esters de cellulose mixtes de deux types d'acides carboxyliques, les copolymères de styrène et d'anhydride maléique, les copolymères de styrène et d'acide acrylique, les terpolymères séquencés styrène-éthylène/butylène-styrène, les terpolymères séquencés styrène-éthylène/propylène-styrène, et les terpolymères d'éthylène, d'acétate de vinyle et d'anhydride maléique, et/ou
- d'au moins une cire choisie parmi la cire d'abeille, la cire d'abeille polyglycérolée, les huiles végétales hydrogénées, la paraffine de point de fusion supérieur à 45 °C, et les cires de silicone.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope.

A titre de cires de silicone, on peut citer par exemple les alkyl-ou alcoxydiméthicones comprenant de 16 à 45 atomes de carbone, comme la béhénoxydiméthicone et les alkylesters de diméthiconol en C₁₆₋₄₅ comme le diméthiconol béhénate.

Elle a en outre pour objet un procédé de fabrication de microcapsules à coeur aqueux et à enveloppe polymérique et/ou cireuse décrites ci-dessus par émulsification multiple-évaporation de solvant.

Les microcapsules dans les compositions selon la présente invention permettent d'obtenir des taux d'encapsulation de principes actifs hydrosolubles au moins égaux à 70 %, voire supérieurs à 95 %, par rapport au poids de l'actif mis en oeuvre.

Elles présentent l'avantage de limiter la fuite des principes actifs hydrophiles encapsulés.

Dans un mode de réalisation de l'invention, l'enveloppe est formée par un ou plusieurs des polymères énumérés ci-dessus associés à au moins une cire choisie parmi celles indiquées ci-dessus.

Ces cires sont dissoutes conjointement avec les polymères organosolubles utilisés selon la présente invention dans le solvant organique avant la préparation de l'émulsion primaire.

L'actif à encapsuler peut être n'importe quelle molécule hydrosoluble ayant une activité cosmétique ou dermatologique. On peut citer à titre d'exemple :
- les agents anti-radicaux libres et/ou détoxifiants tels que l'acide ascorbique et les dérivés de celui-ci comme l'ascorbylphosphate de magnésium, les dérivés de la cystéine comme par exemple la N-acétylcystéine, les protéines, les peptides et leurs dérivés, l'ubiquinone et le cytochrome C,
- les agents kératolytiques tels que les α-hydroxyacides, les β-hydroxyacides et les α-cétoacides comme l'acide salicylique et ses dérivés,
- les accélérateurs de bronzage tels que les dérivés de tyrosine,
- les actifs dépigmentants tels que l'acide kojique, l'arbutine et les dérivés de ceux-ci,
- les filtres UV tels que les filtres à fonction acide sulfonique, en particulier l'acide 2-phénylbenzimidazole-5-sulfonique, la sulisobenzone et l'acide benzène-1,4-di(3-méthylidène-10-camphosulfonique),
- les actifs autobronzants tels que la dihydroxyacétone et les indoles,
- les liporégulateurs tels que la caféine et la théophylline,
- les agents hydratants tels que le sorbitol, le xylitol, l'urée et l'ADN végétal,
- les agents antipelliculaires tels que la piroctone olamine et les dérivés de pyridinethione,
- les azurants optiques tels que les dérivés de stilbène et les colorants tels que les sels de sodium de la tartrazine,
- les colorants naturels extraits de végétaux comme la chlorophylline, ou extraits d'animaux comme le carmin de cochenille, ou le caramel,
ainsi que des mélanges de ces principes actifs.

L'encapsulation dans des microcapsules pour des compositions selon la présente invention est particulièrement intéressante pour des principes actifs instables, sensibles aux différents facteurs physico-chimiques environnants tels que la température, le pH, l'oxygène, la présence d'agents oxydants ou de métaux lourds ou la lumière et le rayonnement UV. L'encapsulation permet en effet de créer un microenvironnement stable mettant les principes actifs à l'abri des agents physico-chimiques contenus dans ou agissant sur la composition cosmétique.

On peut citer notamment à titre d'exemple de principe actif hydrosoluble cosmétique ou dermatologique instable, l'acide ascorbique et ses sels, en particulier les sels de sodium, de potassium, de magnésium ou de calcium.

Pour obtenir un effet cosmétique ou dermatologique, la concentration du principe actif dans la phase aqueuse interne encapsulée est généralement comprise entre 0,1 % et 50 % en poids, de préférence entre 5 et 25 % en poids, par rapport au poids total de la phase aqueuse interne encapsulée. Il peut être souhaitable d'encapsuler des solutions relativement concentrées et la concentration limite supérieure du principe actif est alors imposée par la limite de solubilité de l'actif dans la phase aqueuse interne encapsulée.

Le coeur aqueux des microcapsules, c'est-à-dire la phase aqueuse interne encapsulée de l'émulsion primaire eau-dans-huile dans laquelle se trouve solubilisé le principe actif peut également contenir, à l'état dissous, un ou plusieurs polymères hydrosolubles et/ou un ou plusieurs polyols de faible masse moléculaire. Le rôle des polymères hydrosolubles est de stabiliser l'émulsion primaire et/ou d'éviter la fuite du principe actif à encapsuler hors de la microcapsule. Les polyols ont pour fonction de stabiliser l'actif en diminuant l'activité en eau de la phase aqueuse interne encapsulée.

Les polymères hydrosolubles sont choisis notamment parmi le poly(alcool vinylique), la polyvinylpyrrolidone, la carboxyméthylcellulose, les poly(acide carboxylique) et les dérivés réticulés de ceux-ci, et les gommes naturelles telles que les xanthanes, l'amidon, l'alginate de sodium, les pectines, le chitosane, le guar, le caroube et le carraghénane. Ces polymères hydrosolubles peuvent être présents à raison de 0,01 à 10 %, de préférence à raison de 0,1 à 5 % en poids rapporté au poids total de la phase aqueuse interne encapsulée.

Les polyols de faible masse moléculaire sont choisis par exemple parmi le glycérol et les alkylèneglycols en C₂-C₅, notamment le propylèneglycol et le pentylèneglycol, et peuvent être présents à raison de 10 % à 90 % en poids, de préférence à raison de 10 à 50 % en poids par rapport au poids total de la phase aqueuse interne encapsulée.

La phase aqueuse interne encapsulée de l'émulsion primaire peut en outre contenir un sel destiné à stabiliser l'émulsion primaire. Ce sel, choisi par exemple parmi le chlorure de sodium, le chlorure de potassium, le sulfate de magnésium et le chlorure de magnésium, est habituellement présent en une quantité comprise entre 0,1 et 5 %, et de préférence entre 0,1 et 1 % en poids par rapport au poids total de la phase aqueuse interne encapsulée .

Enfin, la phase aqueuse interne encapsulée de l'émulsion primaire peut également contenir un agent anti-oxydant.

La taille et la structure interne des microcapsules dépend d'un très grand nombre de paramètres liés au procédé de fabrication, tels que la température, la vitesse d'agitation lors de l'émulsification, la nature chimique et les quantités respectives des différents composants hydrosolubles et organosolubles, la quantité d'agents stabilisants etc. L'homme du métier saura faire varier ces différents paramètres pour obtenir la morphologie de microcapsules recherchée.

Les microcapsules peuvent en particulier être univacuolaires ou multivacuolaires, c'est-à-dire l'enveloppe externe peut renfermer un seul compartiment de phase aqueuse ou bien la phase aqueuse interne peut être divisée en une multitude de compartiments séparés par des parois de même nature chimique que l'enveloppe externe. Ce phénomène se produit généralement lorsque l'émulsion multiple est particulièrement stable et donne d'excellents résultats d'encapsulation.

Le rapport en poids de la phase aqueuse interne encapsulée formant le coeur des microcapsules de la présente invention à la paroi de celles-ci (polymère et/ou cire) est généralement compris entre 0,1/1 et 50/1 et de préférence entre 0,5/1 et 10/1.

Les microcapsules dans les compositions de la présente invention ont généralement un diamètre moyen compris entre 1 µm et 1000 µm et plus particulièrement entre 1 et 50 µm.

La concentration en polymère et/ou cire dans la composition cosmétique est notamment comprise entre 0,5 et 10 % en poids et préférentiellement entre 1 et 5 % en poids.

Les compositions cosmétiques ou dermatologiques selon la présente invention peuvent contenir en outre des adjuvants couramment utilisés dans le domaine cosmétique ou dermatologique tels que des corps gras, de la vaseline, des agents régulateurs de pH, des parfums, des conservateurs, des agents épaississants ou gélifiants, des colorants, des agents anti-mousse ou des agents séquestrants.

Bien entendu l'homme de métier veillera à choisir ces éventuels composés complémentaires et leur quantité de manière à ce que les propriétés avantageuses attachées intrinsèquement à la composition cosmétique ou dermatologique selon l'invention ne soient pas, ou substantiellement pas altérées par l'adjonction envisagée.

Les compositions selon l'invention peuvent se présenter par exemple sous forme de sérum, de lotion, de gel aqueux, hydroalcoolique ou huileux, d'émulsion eau-dans-huile ou huile-dans-eau, ou encore sous forme anhydre.
Ces compositions constituent notamment des crèmes de protection, de traitement ou de soin pour le visage, pour les mains ou le corps, des laits corporels de protection ou de soin, des lotions, gels ou mousses pour le soin ou le traitement de la peau, des lotions de nettoyage, des fonds de teint et des crèmes teintées, des rouges à lèvres ou des mascaras. Dans ces derniers cas, la composition renferme en outre des pigments.

La présente invention a également pour objet un procédé de fabrication des microcapsules à coeur aqueux contenant un principe actif hydrosoluble et à enveloppe polymérique et/ou cireuse telles que décrites ci-dessus. Ce procédé est un procédé de microencapsulation par émulsion multiple-évaporation de solvant comportant les étapes successives suivantes :
(a) solubilisation d'au moins un actif cosmétique ou dermatologique dans une phase aqueuse,
(b) émulsification de la solution aqueuse obtenue dans l'étape (a) dans une solution d'au moins un polymère choisi parmi la polycaprolactone, le poly(3-hydroxybutyrate), le poly(éthylène adipate), les esters de cellulose et d'au moins un acide carboxylique en C₁₋₄, de préférence des esters de cellulose mixtes de deux types d'acides carboxyliques, le poly(butylène adipate), les copolymères de styrène et d'anhydride maléique, les copolymères de styrène et d'acide acrylique, les terpolymères séquencés styrène-éthylène/butylène-styrène, les terpolymères séquencés styrène-éthylène/propylène-styrène, et les terpolymères d'éthylène, d'acétate de vinyle et d'anhydride maléique, et/ou d'au moins une cire choisie parmi la cire d'abeille, la cire d'abeille polyglycérolée, les huiles végétales hydrogénées, la paraffine de point de fusion supérieur à 45 °C, et les cires de silicone, dans un solvant organique non miscible à l'eau,
(c) émulsification de l'émulsion primaire eau-dans-huile obtenue dans l'étape (b) dans une solution aqueuse contenant de préférence un agent stabilisant de l'émulsion,
(d) élimination du solvant organique par évaporation donnant une suspension aqueuse de microcapsules.

La nature du solvant organique non miscible à l'eau utilisé dans l'étape (b) est généralement choisie en fonction de son pouvoir solvant vis-à-vis du matériau de la paroi, de sa solubilité dans l'eau qui doit être la plus faible possible et de son point d'ébullition qui est de préférence inférieur à 100 °C. On peut utiliser par exemple le dichlorométhane, le cyclohexane, l'heptane, le chloro-1-butane et l'acétate d'éthyle.

La stabilité de l'émulsion multiple est un facteur déterminant pour l'obtention de bons résultats d'encapsulation. En effet, une stabilité insuffisante de l'émulsion multiple entraînerait un mélange des phases aqueuses interne et externe et une fuite de l'actif hors des vésicules formées. Il est par conséquent fortement recommandé d'ajouter à la phase aqueuse continue de l'étape (c) un agent stabilisant de l'émulsion.
Des agents stabilisants polymériques appropriés sont connus dans la technique et peuvent être choisis par exemple parmi le poly(alcool vinylique), la polyvinylpyrrolidone, les copolymères hydrosolubles styrène-anhydride maléique, la carboxyméthylcellulose, l'amidon, le chitosane et l'acide polyacrylique.

Bien que l'utilisation d'un agent stabilisant polymérique soit préférable, on peut également utiliser à la place de celui-ci des agents tensioactifs hydrosolubles.

En vue d'augmenter la stabilité de l'émulsion multiple, la phase aqueuse continue de celle-ci peut également contenir de 0,1 à 10 % en poids d'un sel minéral choisi par exemple parmi le chlorure de sodium, le chlorure de potassium, le sulfate de magnésium et le chlorure de magnésium.

On pourra également introduire dans la solution de solvant organique de l'étape (b) un agent tensio-actif afin d'améliorer la stabilité de l'émulsion primaire. L'homme du métier saura choisir le composé adéquat, comme par exemple des agents tensio-actifs de HLB (balance hydrophile-lipophile) inférieure à 10, tels que les esters de sorbitane et d'acides gras comme par exemple les polysorbates, les lécithines liposolubles, les monoglycérides d'acides gras, le PEG-30 dipolyhydrostéarate (ARLACEL^{®} P135 de la société ICI), le cétyldiméthicone-copolyol (ABIL^{®} EM90 de la société GOLDSCHMIDT) et le polydiméthylsiloxane oxyéthyléné (DC2-5695^{®} de la société DOW CHEMICAL).

La suspension de microcapsules obtenue après évaporation du solvant peut être utilisée telle quelle ou être incorporée dans une composition cosmétique ou dermatologique.
Si on le souhaite, les microcapsules peuvent également être séparées à partir de la suspension aqueuse obtenue dans l'étape (d) par filtration et on peut les sécher de manière à obtenir une poudre de microcapsules.
Les exemples suivants illustrent l'encapsulation de deux actifs cosmétiques thermosensibles.

### Exemple 1

### Microcapsules de cytochrome C

On prépare une solution aqueuse de cytochrome C (qui peut être obtenu sous la référence C3256 auprès de la société SIGMA) à 0,25 % en poids. On émulsionne 5 ml de cette solution dans 50 ml de dichlorométhane contenant 5 % d'acéto-butyrate de cellulose (CAB-381-05^{®} , Eastman Chemical) à l'aide d'un homogénéiseur de type rotor-stator, pendant 5 minutes en maintenant la température en dessous de 25 °C. Cette émulsion primaire est ensuite dispersée dans 500 ml d'une solution aqueuse contenant 1 % de poly(alcool vinylique) (Rhodoviol 4-125, Rhodia Chimie), à l'aide d'un disperseur Moritz pendant 20 minutes à température ambiante.
Le solvant de la suspension est évaporé à l'aide d'un évaporateur rotatif (Büchi B-480) pendant 5 heures à 40 °C, à une pression de 75 kPa.
On obtient une suspension aqueuse de microcapsules ayant un diamètre moyen de 22 µm.
Le dosage du cytochrome C est effectué par spectrophotométrie UV-visible à une longueur d'onde de 506 nm.
Le taux d'encapsulation, c'est-à-dire le rapport de la concentration d'actif retrouvée à l'intérieur des microcapsules à la concentration totale d'actif, est égal à 97 %. Le rendement de fabrication, c'est-à-dire le rapport de la quantité d'actif récupérée en fin de fabrication à la quantité d'actif mise en jeu au départ, est égal à 100 %.

On conserve la suspension de microcapsules ainsi obtenue pendant 2 mois à une température de 45 °C. A l'issue de cette période de conservation, on mesure la perte en cytochrome C de la suspension et celle d'une solution aqueuse témoin de cytochrome C ayant la même concentration en poids de 0,25 % et qui a été conservée dans les mêmes conditions. Les résultats obtenus sont présentés dans le tableau ci-après.

### Exemple 2

### Microcapsules de chlorophylline

On prépare une solution aqueuse de chlorophylline à 0,1 % en poids. On émulsionne 5 ml de cette solution dans 50 ml de dichlorométhane contenant 5 % de poly(ε-caprolactone) (CAPA640^{®} , Solvay) à l'aide d'un homogénéiseur de type rotor-stator pendant 5 minutes en maintenant la température en dessous de 25 °C. Cette émulsion primaire est ensuite dispersée dans 500 ml d'une solution aqueuse contenant 1 % de poly(alcool vinylique) (Rhodoviol 4-125, Rhodia Chimie), à l'aide d'un disperseur Moritz pendant 20 minutes à température ambiante.
Le solvant de la suspension est ensuite évaporé à l'aide d'un évaporateur rotatif (Büchi B-480) pendant 5 heures à 40 °C, à une pression de 75 kPa.
On obtient une suspension aqueuse de microcapsules ayant un diamètre moyen de 15 µm.
Le dosage de la chlorophylline est effectué par spectrophotométrie UV-visible à une longueur d'onde de 405 nm.
Le taux d'encapsulation est égal à 90 % et le rendement de fabrication égal à 100 %.

On conserve la suspension de microcapsules ainsi obtenue pendant 2 mois à une température de 45 °C. A l'issue de cette période de conservation, on mesure la perte en chlorophylline de la suspension et celle d'une solution aqueuse témoin de chlorophylline ayant la même concentration en poids de 0,1 % et qui a été conservée dans les mêmes conditions. Les résultats obtenus sont présentés dans le tableau ci-après.

### Exemple 3

### Microcapsules d'acide ascorbique

On prépare une solution aqueuse d'acide ascorbique à 5 % en poids, à pH 6. On émulsionne 5 ml de cette solution dans 50 ml de dichlorométhane contenant 5 % d'acétopropionate de cellulose (CAP-482-0.5^{®}, Eastman Chemical) à l'aide d'un homogénéiseur de type rotor-stator, pendant 5 minutes en maintenant la température en dessous de 25 °C. Cette émulsion primaire est ensuite dispersée dans 500 ml d'une solution aqueuse contenant 1 % de poly(alcool vinylique) (Airvol 203^{®} , Air Products) et 7 % de chlorure de sodium, à l'aide d'un disperseur Moritz pendant 20 minutes à température ambiante.
Le solvant de la suspension est ensuite évaporé à l'aide d'un évaporateur rotatif (Büchi B-480) pendant 5 heures à 40 °C, à une pression de 75 kPa.
La taille moyenne des microcapsules obtenues est de 20 µm.
Le dosage de l'acide ascorbique est effectué par HPLC dans un tampon phosphate à 0,1 mole/litre à pH 2,1, à une longueur d'onde de détection de 257 nm.
Le taux d'encapsulation est de 85 % et le rendement de fabrication de 100 %.

On conserve la suspension de microcapsules ainsi obtenue pendant 2 mois à une température de 45 °C. A l'issue de cette période de conservation, on mesure la perte en acide ascorbique de la suspension et celle d'une solution aqueuse témoin d'acide ascorbique ayant la même concentration en poids de 5 % et également un pH de 6, et qui a été conservée dans les mêmes conditions. Les résultats obtenus sont présentés dans le tableau ci-après.

### Exemple 4 (comparatif)

On prépare une solution aqueuse d'acide ascorbique à 10 % en poids, à pH 6. On émulsionne 5 ml de cette solution dans 50 ml de dichlorométhane contenant 5 % de polystyrène (PM 50 000, Polysciences) à l'aide d'un homogénéiseur de type rotor-stator, pendant 5 minutes en maintenant la température en dessous de 25 °C. Cette émulsion primaire est ensuite dispersée dans 500 ml d'une solution aqueuse contenant 1 % de poly(alcool vinylique) (Airvol 230^{®}, AirProducts) et 7 % de chlorure de sodium, à l'aide d'un disperseur Moritz pendant 20 minutes à température ambiante.
Le solvant de la suspension est ensuite évaporé à l'aide d'un évaporateur rotatif (Büchi B-480) pendant 5 heures à 40 °C, à une pression de 75 kPa.
La taille moyenne des particules obtenues est de 15 µm.
Le dosage de l'acide ascorbique est effectué par HPLC dans un tampon phosphate à 0,1 mole/litre à pH 2,1, à une longueur d'onde de détection de 257 nm.
Le taux d'encapsulation est de 0 % avec un rendement de fabrication de 100 %.

### Exemple 5 (comparatif)

On prépare une solution aqueuse d'acide ascorbique à 10 % en poids, à pH 6. On émulsionne 5 ml de cette solution dans 50 ml de dichlorométhane contenant 5 % d'éthylcellulose (Ethocel^{®} Standard FP 7 Premium, Dow Chemical) à l'aide d'un homogénéiseur de type rotor-stator pendant 5 minutes en maintenant la température en dessous de 25 °C. Cette émulsion primaire est ensuite dispersée dans 500 ml d'une solution aqueuse contenant 1 % de poly(alcool vinylique) (Airvol 203^{®} , AirProducts) et 7,5 % de chlorure de sodium à l'aide d'un disperseur Moritz pendant 20 minutes à température ambiante.
Le solvant de la suspension est ensuite évaporé à l'aide d'un évaporateur rotatif (Büchi B-480) pendant 5 heures à 40 °C, à une pression de 75 kPa.
La taille moyenne des microcapsules obtenues est de 18 µm.
Le dosage de l'acide ascorbique est effectué par HPLC dans un tampon phosphate à 0,1 mole/litre à pH 2,1, à une longueur d'onde de détection de 257 nm.
Le taux d'encapsulation est de 17 % avec un rendement de fabrication de 100 %.

**Tableau**

| | taux d'encapsulation | Perte en actif après 2 mois de conservation à 45°C | |
|---|---|---|---|
| | | actif encapsulé | témoin non encapsulé |
| Exemple 1 | 97 % | 0 % | 60 % |
| Exemple 2 | 90 % | 10 % | 60 % |
| Exemple 3 | 85 % | 20 % | 40 % |
| Exemple 4 (comparatif) | 0 % | - | - |
| Exemple 5 (comparatif) | 17 % | - | - |

Ces essais comparatifs montrent l'intérêt de l'utilisation d'un ester de cellulose (Exemples 1 et 3) ou de polycaprolactone (Exemple 2) à la place du polystyrène (Exemple comparatif 4) ou d'un éther de cellulose (Exemple comparatif 5) pour l'obtention d'un taux d'encapsulation élevé. De plus, on constate que les microcapsules utilisées dans les compositions de l'invention permettent d'obtenir une meilleure stabilité chimique des actifs cosmétiques qui y sont inclus.

### Exemple 6

### Microcapsules de tartrazine

On prépare une solution aqueuse de tartrazine à 0,1 % en poids. On émulsionne 5 ml de cette solution dans 50 ml de dichlorométhane contenant 5 % d'un mélange 90/10 en poids d'acétopropionate de cellulose (CAP-482-0.5^{®}, Eastman Chemical) et de cire d'abeille, à l'aide d'un homogénéiseur de type rotor-stator, pendant 5 minutes en maintenant la température en dessous de 25 °C. Cette émulsion primaire est ensuite dispersée dans 500 ml d'une solution aqueuse contenant 1 % de poly(alcool vinylique) (Airvol 203^{®} , AirProducts) à l'aide d'un disperseur Moritz pendant 20 minutes à température ambiante.
Le solvant de la suspension est ensuite évaporé à l'aide d'un évaporateur rotatif (Büchi B-480) pendant 5 heures à 40 °C, à une pression de 75 kPa.
La taille moyenne des microcapsules obtenues est de 25 µm.
Le taux d'encapsulation est de 98 % avec un rendement de fabrication de 100 %.

### Exemple 7

### Crème de jour contenant les microcapsules de l'Exemple 3

| *Phase A* | | |
|---|---|---|
| | alcool cétylique | 4 g |
| | tristéarate de sorbitane | 0,9 g |
| | stéarate de polyéthylèneglycol | 2 g |
| | stéarate de glycérol | 3 g |
| | myristate de myristyle | 2 g |
| | palmitate d'octyle | 4,5 g |
| | Parsol MCX^{®} (vendu par Hoffman-Laroche) | 3 g |
| | cyclopentasiloxane | 5 g |
| | conservateur | 0,1 g |

| *Phase B* | | |
|---|---|---|
| | eau déminéralisée | 60,3 g |
| | conservateur | 0,15 g |
| | séquestrant | 0,05 g |

| *Phase C* | | |
|---|---|---|
| | poudre de microcapsules de l'exemple 3 | 15 g |

On chauffe rapidement les phases A et B jusqu'à une température de 75 °C et on les mélange à l'aide d'un agitateur de type MORITZ (Turbolab 2100). On ramène ensuite l'émulsion à température ambiante en maintenant l'agitation, puis on y introduit lentement la poudre de microcapsules (phase C), sous faible agitation à l'aide d'un agitateur à pales du type HEIDOLPH (RZR 2040).

## Revendications

1. Composition cosmétique sous la forme d'un gel aqueux, d'une émulsion eau-dans-huile ou d'une émulsion huile-dans-eau, comprenant, dans un support physiologiquement acceptable, des microcapsules à coeur aqueux contenant au moins un principe actif cosmétique, hydrosoluble, instable, sensible aux conditions physico-chimiques environnantes, et à enveloppe polymérique et/ou cireuse, **caractérisée par le fait que** ladite enveloppe est constituée
- d'au moins un polymère choisi parmi la polycaprolactone, le poly(3-hydroxybutyrate), le poly(éthylène adipate), le poly(butylène adipate), les esters de cellulose et d'au moins un acide carboxylique en C₁₋₄, de préférence des esters de cellulose mixtes de deux types d'acides carboxyliques, les copolymères de styrène et d'anhydride maléique, les copolymères de styrène et d'acide acrylique, les terpolymères séquencés styrène-éthylène/butylène-styrène, les terpolymères séquencés styrène-éthylène/propylène-styrène, et les terpolymères d'éthylène, d'acétate de vinyle et d'anhydride maléique, et/ou
- d'au moins une cire choisie parmi la cire d'abeille, la cire d'abeille polyglycérolée, les huiles végétales hydrogénées, la paraffine de point de fusion supérieur à 45 °C et les cires de silicone.

2. Composition selon la revendication 1, **caractérisée par le fait que** ladite enveloppe des microcapsules est constituée d'au moins un desdits polymères et d'au moins une desdites cires.

3. Composition selon l'une quelconque des revendications précédentes, **caractérisée par le fait que** le principe actif instable des microcapsules est sensible aux conditions physico-chimiques environnantes telles que la température, le pH, l'oxygène, la présence d'agents oxydants ou de métaux lourds, la lumière ou le rayonnement UV.

4. Composition selon la revendication 3, **caractérisée par le fait que** ledit principe actif est choisi parmi les agents anti-radicaux libres et/ou détoxifiants, les agents kératolytiques, les accélérateurs de bronzage, les actifs dépigmentants, les filtres UV, les actifs autobronzants, les liporégulateurs, les agents hydratants, les agents antipelliculaires, les azurants optiques ou un mélange de ceux-ci.

5. Composition selon la revendication 4, **caractérisée par le fait que** ledit principe actif est l'acide ascorbique ou les sels de celui-ci.

6. Composition selon l'une quelconque des revendications précédentes, **caractérisée par le fait que** la concentration dudit principe actif dans la phase aqueuse interne encapsulée est comprise entre 0,1 % et 50% en poids, de préférence entre 5 et 25% en poids par rapport au poids total de la phase aqueuse interne encapsulée.

7. Composition selon l'une quelconque des revendications précédentes, **caractérisée par le fait que** le coeur aqueux des microcapsules contient en outre un ou plusieurs polyols de faible masse moléculaire.

8. Composition selon l'une quelconque des revendications précédentes, **caractérisée par le fait que** le coeur aqueux des microcapsules contient en outre un ou plusieurs polymères hydrosolubles.

9. Composition selon l'une quelconque des revendications précédentes, **caractérisée par le fait que** le rapport en poids de la phase aqueuse interne encapsulée formant le coeur des microcapsules à la paroi de celles-ci est compris entre 0,1/1 et 50/1 de préférence entre 0,5/1 et 10/1.

10. Composition selon l'une quelconque des revendications précédentes, **caractérisée par le fait que** les cires de silicone sont choisies parmi les alkyl- ou alcoxydiméthicones comprenant de 16 à 45 atomes de carbone, comme la béhénoxydiméthicone et les alkylesters de diméthiconol en C₁₆₋₄₅ comme le diméthiconol béhénate.

11. Composition selon l'une quelconque des revendications précédentes, **caractérisée par le fait que** les microcapsules ont un diamètre moyen compris entre 1 µm et 1000 µm, de préférence entre 1 et 50 µm.

12. Composition selon l'une des revendications précédentes, **caractérisée par le fait que** la concentration en polymère et/ou en cire dans la composition cosmétique est comprise entre 0,5 et 10 % en poids et préférentiellement entre 1 et 5% en poids.

13. Procédé de fabrication de microcapsules définies à l'une quelconque des revendications 1 à 12, comportant les étapes successives suivantes consistant
(a) à solubiliser au moins un actif cosmétique dans une phase aqueuse,
(b) à émulsifier la solution aqueuse obtenue dans l'étape (a) dans une solution d'au moins un polymère et/ou d'au moins une cire, dans un solvant organique non miscible à l'eau,
(c) à émulsifier l'émulsion primaire eau-dans-huile obtenue dans l'étape (b) dans une solution aqueuse contenant de préférence un agent stabilisant de l'émulsion,
(d) à éliminer le solvant organique par évaporation de manière à obtenir une suspension aqueuse de microcapsules, **caractérisé par le fait que** lesdits polymères utilisés dans l'étape (b) sont choisis dans le groupe formé par la polycaprolactone, le poly(3-hydroxybutyrate), le poly(éthylène adipate), le poly(butylène adipate), les esters de cellulose et d'au moins un acide carboxylique en C₁₋₄, de préférence des esters de cellulose mixtes de deux types d'acides carboxyliques, les copolymères de styrène et d'anhydride maléique, les copolymères de styrène et d'acide acrylique, les terpolymères séquencés styrène-éthylène/butylène-styrène, les terpolymères séquencés styrène-éthylène/propylène-styrène, et les terpolymères d'éthylène, d'acétate de vinyle et d'anhydride maléique, et lesdites cires sont choisies parmi la cire d'abeille, la cire d'abeille polyglycérolée, les huiles végétales hydrogénées, la paraffine de point de fusion supérieur à 45 °C, et les cires de silicone.

14. Procédé selon la revendication 13, **caractérisé par le fait que** le solvant organique non miscible à l'eau utilisé dans l'étape (b) est choisi parmi le dichlorométhane, le cyclohexane, l'heptane, le chloro-1-butane et l'acétate d'éthyle.

15. Procédé selon la revendication 13 ou 14, **caractérisé par le fait que** ledit agent stabilisant de l'émulsion utilisé dans l'étape (c) est choisi parmi le poly(alcool vinylique), la polyvinylpyrrolidone, les copolymères hydrosolubles styrène-anhydride maléique, la carboxyméthylcellulose, l'amidon, le chitosane et l'acide polyacrylique.

## Claims

1. Cosmetic composition in the form of an aqueous gel, of a water-in-oil emulsion or of an oil-in-water emulsion, comprising, in a physiologically acceptable support, microcapsules with an aqueous core containing at least one unstable water-soluble cosmetic active principle, which is sensitive to the surrounding physicochemical conditions, and with a polymeric and/or waxy envelope, **characterized in that** the said envelope consists of
- at least one polymer chosen from polycaprolactone, poly(3-hydroxybutyrate), poly(ethylene adipate), poly(butylene adipate), esters of cellulose and of at least one C₁₋₄ carboxylic acid, preferably mixed cellulose esters of two types of carboxylic acid, copolymers of styrene and of maleic anhydride, copolymers of styrene and of acrylic acid, styrene-ethylene/butylene-styrene block terpolymers, styrene-ethylene/propylene-styrene block terpolymers and terpolymers of ethylene, of vinyl acetate and of maleic anhydride, and/or
- at least one wax chosen from beeswax, polyglycerolated beeswax, hydrogenated plant oils, paraffin with a melting point of greater than 45°C and silicone waxes.

2. Composition according to Claim 1, **characterized in that** the said envelope of the microcapsules consists of at least one of the said polymers and of at least one of the said waxes.

3. Composition according to either of the preceding claims, **characterized in that** the unstable active principle of the microcapsules is sensitive to the surrounding physicochemical conditions such as the temperature, the pH, oxygen, the presence of oxidizing agents or heavy metals, light or UV radiation.

4. Composition according to Claim 3, **characterized in that** the said active principle is chosen from free-radical scavengers and/or detoxifying agents, keratolytic agents, tanning accelerators, depigmenting active agents, UV-screening agents, self-tanning active agents, liporegulators, moisturizers, antidandruff agents and optical brighteners, or a mixture thereof.

5. Composition according to Claim 4, **characterized in that** the said active principle is ascorbic acid or salts thereof.

6. Composition according to any one of the preceding claims, **characterized in that** the concentration of the said active principle in the encapsulated inner aqueous phase is between 0.1% and 50% by weight and preferably between 5% and 25% by weight relative to the total weight of the encapsulated inner aqueous phase.

7. Composition according to any one of the preceding claims, **characterized in that** the aqueous core of the microcapsules also contains one or more polyols of low molecular mass.

8. Composition according to any one of the preceding claims, **characterized in that** the aqueous core of the microcapsules also contains one or more water-soluble polymers.

9. Composition according to any one of the preceding claims, **characterized in that** the weight ratio of the encapsulated inner aqueous phase forming the core of the microcapsules to the wall thereof is between 0.1/l and 50/l and preferably between 0.5/l and 10/l.

10. Composition according to any one of the preceding claims, **characterized in that** the silicone waxes are chosen from alkyl or alkoxy dimethicones containing from 16 to 45 carbon atoms, for instance behenoxy dimethicone and C₁₆₋₄₅ dimethiconol alkyl esters, for instance dimethiconol behenate.

11. Composition according to any one of the preceding claims, **characterized in that** the microcapsules have a mean diameter of between 1 µm and 1000 µm and preferably between 1 and 50 µm.

12. Composition according to one of the preceding claims, **characterized in that** the concentration of polymer and/or wax in the cosmetic composition is between 0.5% and 10% by weight and preferentially between 1% and 5% by weight.

13. Process for manufacturing microcapsules defined in any one of Claims 1 to 12, comprising the following successive steps consisting in:
(a) dissolving at least one cosmetic active agent in an aqueous phase,
(b) emulsifying the aqueous solution obtained in step (a) in a solution of at least one polymer and/or of at least one wax, in a water-immiscible organic solvent,
(c) emulsifying the water-in-oil primary emulsion obtained in step (b) in an aqueous solution preferably containing an emulsion stabilizer,
(d) removing the organic solvent by evaporation so as to obtain an aqueous suspension of microcapsules, **characterized in that** the said polymers used in step (b) are chosen from the group formed by polycaprolactone, poly(3-hydroxybutyrate), poly(ethylene adipate), poly (butylene adipate), esters of cellulose and of at least one C₁₋₄ carboxylic acid, preferably mixed cellulose esters of two types of carboxylic acid, copolymers of styrene and of maleic anhydride, copolymers of styrene and of acrylic acid, styrene-ethylene/butylene-styrene block terpolymers, styrene-ethylene/propylene-styrene block terpolymers and terpolymers of ethylene, of vinyl acetate and of maleic anhydride, and the said waxes are chosen from beeswax, polyglycerolated beeswax, hydrogenated plant oils, paraffin with a melting point of greater than 45°C, and silicone waxes.

14. Process according to Claim 13, **characterized in that** the water-immiscible organic solvent used in step (b) is chosen from dichloromethane, cyclohexane, heptane, 1-chlorobutane and ethyl acetate.

15. Process according to Claim 13 or 14, **characterized in that** the said emulsion stabilizer used in step (c) is chosen from poly(vinyl alcohol), polyvinylpyrrolidone, styrene-maleic anhydride water-soluble copolymers, carboxymethylcellulose, starch, chitosan and polyacrylic acid.

## Patentansprüche

1. Kosmetische Zusammensetzung in der Form eines wässrigen Gels, einer Wasser-in-Öl-Emulsion oder einer Öl-in-Wasser-Emulsion, die in einem physiologisch akzeptablen Träger Mikrokapseln mit einem wässrigen Kern, der mindestens einen gegenüber den physikalisch-chemischen Bedingungen der Umgebung empfindlichen, instabilen, wasserlöslichen kosmetischen Wirkstoff enthält, und mit einer Polymerhülle und/oder Wachshülle enthält, **dadurch gekennzeichnet, dass** die Hülle besteht aus:
- mindestens einem Polymer, das unter Polycaprolacton, Poly(3-hydroxybutyrat), Poly(ethylenadipat), Poly(butylenadipat), Estern von Cellulose und mindestens einer C₁₋₄-Carbonsäure und vorzugsweise gemischten Celluloseestern von zwei Arten von Carbonsäuren, Copolymeren von Styrol und Maleinsäureanhydrid, Copolymeren von Styrol und Acrylsäure, Styrol-Ethylen/Butylen-Styrol-Sequenzterpolymeren, Styrol-Ethylen/Propylen-Styrol-Sequenzterpolymeren und Terpolymeren von Ethylen, Vinylacetat und Maleinsäureanhydrid ausgewählt ist, und/oder
- mindestens einem Wachs, das unter Bienenwachs, mehrfach mit Glycerin verethertem Bienenwachs, hydrierten pflanzlichen Ölen, Paraffin mit einem Schmelzpunkt über 45 °C und Siliconwachsen ausgewählt ist.

2. Zusammensetzung nach Anspruch 1, **dadurch gekennzeichnet, dass** die Hülle der Mikrokapseln aus zumindest einem dieser Polymere und zumindest einem dieser Wachse besteht.

3. Zusammensetzung nach einem der vorhergehenden Ansprüche, **dadurch gekennzeichnet, dass** der instabile Wirkstoff der Mikrokapseln gegenüber den physikalisch-chemischen Bedingungen in der Umgebung empfindlich ist, wie Temperatur, pH-Wert, Sauerstoff, der Gegenwart von Oxidantien oder Schwermetallen, Licht oder UV-Strahlung.

4. Zusammensetzung nach Anspruch 3, **dadurch gekennzeichnet, dass** der Wirkstoff unter den Radikalfängern für freie Radikale und/oder Entgiftungsmitteln, Keratolytika, Bräunungsbeschleunigern, depigmentierenden Wirkstoffen, UV-Filtern, Selbstbräunungsmitteln, Liporegulatoren, Hydratisierungsmitteln, Antischuppenmitteln, optischen Aufhellern oder deren Gemischen ausgewählt ist.

5. Zusammensetzung nach Anspruch 4, **dadurch gekennzeichnet, dass** es sich bei dem Wirkstoff um Ascorbinsäure oder ihre Salze handelt.

6. Zusammensetzung nach einem der vorhergehenden Ansprüche, **dadurch gekennzeichnet, dass** die Konzentration des Wirkstoffes in der eingekapselten inneren wässrigen Phase im Bereich von 0,1 bis 50 Gew.-% und vorzugsweise 5 bis 25 Gew.-%, bezogen auf das Gesamtgewicht der eingekapselten inneren wässrigen Phase liegt.

7. Zusammensetzung nach einem der vorhergehenden Ansprüche, **dadurch gekennzeichnet, dass** der wässrige Kern der Mikrokapseln ferner ein oder mehrere Polyole von geringer Molmasse enthält.

8. Zusammensetzung nach einem der vorhergehenden Ansprüche, **dadurch gekennzeichnet, dass** der wässrige Kern der Mikrokapseln ferner ein oder mehrere wasserlösliche Polymere enthält.

9. Zusammensetzung nach einem der vorhergehenden Ansprüche, **dadurch gekennzeichnet, dass** das Gewichtsverhältnis der eingekapselten inneren wässrigen Phase, die den Kern der Mikrokapseln bildet, und der Wand der Mikrokapseln im Bereich von 0,1/l bis 50/l und vorzugsweise 0,5/l bis 10/l liegt.

10. Zusammensetzung nach einem der vorhergehenden Ansprüche, **dadurch gekennzeichnet, dass** die Siliconwachse unter den Alkyl- oder Alkoxydimethiconen mit 16 bis 45 Kohlenstoffatomen, wie Behenoxydimethicon, und Alkylestern von Dimethiconol mit 16 bis 45 Kohlenstoffatomen, wie Dimethiconolbehenat, ausgewählt sind.

11. Zusammensetzung nach einem der vorhergehenden Ansprüche, **dadurch gekennzeichnet, dass** die Mikrokapseln einen mittleren Durchmesser von 1 bis 1 000 µm und vorzugsweise 1 bis 50 µm aufweisen.

12. Zusammensetzung nach einem der vorhergehenden Ansprüche, **dadurch gekennzeichnet, dass** die Konzentration des Polymers und/oder des Wachses in der kosmetischen Zusammensetzung im Bereich von 0,5 bis 10 Gew.-% und vorzugsweise 1 bis 5 Gew.-% liegt.

13. Verfahren zur Herstellung von Mikrokapseln nach einem der Ansprüche 1 bis 12, das die folgenden aufeinander folgenden Schritte umfasst:
(a) Solubilisieren mindestens eines kosmetischen Wirkstoffes in einer wässrigen Phase,
(b) Emulgieren der in Schritt (a) erhaltenen wässrigen Lösung in einer Lösung von zumindest einem Polymer und/oder zumindest einem Wachs in einem nicht mit Wasser mischbaren organischen Lösungsmittel,
(c) Emulgieren der in Schritt (b) erhaltenen Wasser-in-Öl-Primäremulsion in einer wässrigen Lösung, die vorzugsweise ein Stabilisierungsmittel für die Emulsion enthält,
(d) Entfernen des organischen Lösungsmittels durch Verdampfen, sodass eine wässrige Suspension von Mikrokapseln erhalten wird, **dadurch gekennzeichnet, dass** die in Schritt (b) verwendeten Polymere ausgewählt sind unter: Polycaprolacton, Poly(3-hydroxybutyrat), Poly(ethylenadipat), Poly(butylenadipat), Estern von Cellulose und mindestens einer C₁₋₄₋Carbonsäure und vorzugsweise gemischten Celluloseestern von zwei Arten von Carbonsäuren, Copolymeren von Styrol und Maleinsäureanhydrid, Copolymeren von Styrol und Acrylsäure, Styrol-Ethylen/Butylen-Styrol-Sequenzterpolymeren, Styrol-Ethylen/Propylen-Styrol-Sequenzterpolymeren und Terpolymeren von Ethylen, Vinylacetat und Maleinsäureanhydrid, und die Wachse ausgewählt sind unter: Bienenwachs, mehrfach mit Glycerin verethertem Bienenwachs, hydrierten pflanzlichen Ölen, Paraffin mit einem Schmelzpunkt über 45 °C und Siliconwachsen.

14. Verfahren nach Anspruch 13, **dadurch gekennzeichnet, dass** das in Schritt (b) verwendete, nicht mit Wasser mischbare organische Lösungsmittel unter Dichlormethan, Cyclohexan, Heptan, 1-Chlorbutan und Ethylacetat ausgewählt ist.

15. Verfahren nach Anspruch 13 oder 14, **dadurch gekennzeichnet, dass** das Stabilisierungsmittel für die Emulsion, das in Schritt (c) verwendet wird, unter Poly(vinylalkohol), Polyvinylpyrrolidon, wasserlöslichen Styrol-Maleinsäureanhydrid-Copolymeren, Carboxymethylcellulose, Stärke, Chitosan und Polyacrylsäure ausgewählt ist.
